(19) Europäisches Patentamt
European Patent Office
Office européen des brevets

(11) Publication number: 0 162 193
A1

# (12) EUROPEAN PATENT APPLICATION

(21) Application number: 85101420.9

(22) Date of filing: 28.01.82

(51) Int. Cl.[4]: C 07 D 499/00
C 07 D 471/04, C 07 D 487/04
C 07 D 498/04, A 61 K 31/40
A 61 K 31/415, A 61 K 31/42
A 61 K 31/43, A 61 K 31/435
//(C07D471/04, 221:00, 205:00),
(C07D487/04, 209:00, 205:00),
(C07D487/04, 235:00, 205:00),
(C07D498/04, 263:00, 205:00)

(30) Priority: 02.02.81 US 230774

(43) Date of publication of application:
27.11.85 Bulletin 85/48

(84) Designated Contracting States:
AT BE CH DE FR GB IT LI NL SE

(60) Publication number of the earlier application in accordance with Art. 76 EPC: 0 058 317

(71) Applicant: SCHERING CORPORATION
2000 Galloping Hill Road
Kenilworth, New Jersey 07033(US)

(72) Inventor: Afonso, Adriano
10 Woodmere Road
West Caldwell, N.J. 07006(US)

(72) Inventor: Hon, Frank
596 Paramus Road
Paramus, N.J. 07652(US)

(74) Representative: Ritter, Stephen David et al,
Mathys & Squire 10 Fleet Street
London EC4Y 1AY(GB)

## (54) Novel 2-penem-type compounds and pharmaceutical compositions contaning them.

(57) Compounds of the general formula:

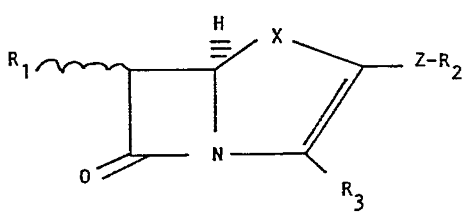

I

in which $R_1$, $R_2$, $R_3$, X and Z are as defined herein, and their pharmaceutically acceptable salts;

especially those subject to the following provisos:

(i) when X is S, Z is O or $=NR_7$ ($R_7$ being as defined herein);

(ii) when X is O, Z is O, S or $=NR_7$;

(iii) when X is $-CH_2-$, Z is $=NR_7$;

(except that when $R_7$ is H or $C_1$-$C_6$ alkyl and $R_2$ is $C_1$-$C_6$ alkyl, then $R_1$ cannot be acylamino;

(iv) when X is $(CH_2)_2$, Z is $=NR_7$;

and their pharmaceutically acceptable salts. These compounds are antibacterial agents which can be incorporated into pharmaceutical compositions or used as intermediates in the preparation of such agents.

EP 0 162 193 A1

Croydon Printing Company Ltd.

NOVEL 2-PENEM-TYPE COMPOUNDS AND PHARMACEUTICAL COMPOSITIONS CONTAINING THEM

This invention relates to novel 2-penem-type compounds having the general formula

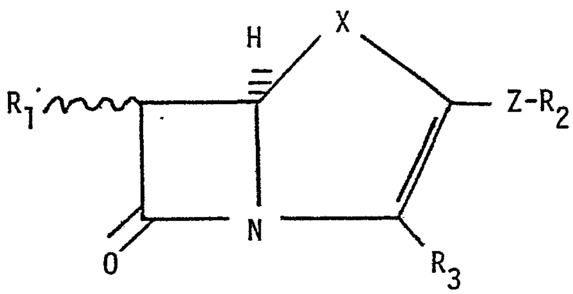

I

in which $R_1$ is hydrogen, $C_1$-$C_6$ alkyl, acylamino or $R_4\overset{OR_5}{\text{-CH-}}$, wherein $R_4$ is hydrogen, $C_1$-$C_6$ alkyl, aryl, phenyl substituted by $C_1$-$C_6$ alkyl, by $C_1$-$C_6$ alkoxy or by halogen, or heteroaryl and $R_5$ is hydrogen or an hydroxy protecting group;

$R_2$ is $C_1$-$C_6$ alkyl, aryl, phenyl substituted by $C_1$-$C_6$ alkyl, by $C_1$-$C_6$ alkoxy or by halogen, $C_1$-$C_6$ alkyl substituted by one or more aryl groups, aminoalkyl, N-protected aminoalkyl, hydroxyalkyl, O-protected hydroxyalkyl, an optionally esterfied, optionally N-protected $\alpha$-amino acid residue or an optionally esterfied carboxyalkyl group;

$R_3$ is nitrile, tetrazolyl or $-COOR_6$, wherein $R_6$ is hydrogen, $-Alk-C(Hal)_3$ (in which Hal is halogen and Alk represents a $C_1$-$C_6$ alkylene radical), $C_1$-$C_6$ alkyl, aryl, phenyl substituted by $C_1$-$C_6$ alkyl, by $C_1$-$C_6$ alkoxy or by halogen, an allylic group, an ester group which can be metabolically removed in vivo, or a carboxy protecting group;

X and Z are independently sulfur, oxygen, $-CH_2-$, $-CH_2-CH_2-$, or the radical $=NR_7$ in which $R_7$ is hydrogen, acyl, $C_1$-$C_6$ alkyl, cycloalkyl containing 3 to 6 carbon atoms, aryl, phenyl substituted by $C_1$-$C_6$ alkyl, by $C_1$-$C_6$

alkoxy or by halogen, or an N-protecting group; and wherein in the above definitions heteroaryl means an aryl group having a heteroatom in the ring and optionally having 1 to 3 $C_1$-$C_6$ alkyl substituents, and acyl refers to acyl groups derived from carboxylic acids; and the pharmaceutically acceptable salts thereof.

The preferred compounds of the invention correspond to above formula I, subject to the following provisos:

(i) when X is S, Z is O or $=NR_7$ ($R_7$ being as defined above);

(ii) when X is O, Z is O, S or $=NR_7$;

(iii) when X is $-CH_2-$, Z is $=NR_7$ (except that when $R_7$ is H or $C_1$-$C_6$ alkyl and $R_2$ is $C_1$-$C_6$ alkyl, then $R_1$ cannot be acylamino);

(iv) when X is $(CH_2)_2$, Z is $=NR_7$;

including the pharmaceutically acceptable salts thereof. More preferably X is sulfur, $-CH_2-$ or $=NR_7$, and is most preferably $=NR_7$.

For convenience the compounds of formula I wherein X is $-CH_2-$ maybe identified as carbapenems; those in which X is $-CH_2-CH_2-$ as carbacephams, and those in which X is sulfur, oxygen and $=NR_7$ respectively as penems, oxapenems and azapenems.

The compounds of the invention can be prepared by the process of European patent application No. 82100585.7 (Publ.No. 58317), of which the present application is a divisional application.

Some compounds of formula I in which X is sulfur and Z is S or $-(CH_2)-$, and from which any O- and N-protecting groups have been removed, have been proposed as antibacterial agents, and are disclosed, together with process for their production, in for example, European Published Patent Applications Nos: 13,662 and 3960.

The process of the parent application, EP-A-58317, comprises the preparation of a compound of formula I by reacting a compound of the general formula

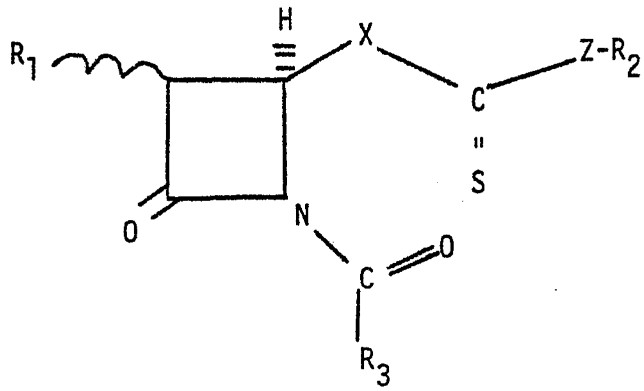

II

in which $R_1$, $R_2$, $R_3$, X and Z are as defined above, wherein $R_5$ is a hydroxy protecting group, $R_7$ is not hydrogen, any carboxy group in substituent $R_2$ is esterified, and $R_6$ is not hydrogen; with an organo-substituted derivative of phosphorous acid;

and if required or desired, subjecting the resulting compound, prior to or subsequent to isolation and any separation into its stereochemical isomers, if a mixture of isomers of compounds of formula II was subjected to the foregoing reaction, to one or more of the following operations:

a) removal of one or more protecting group;
b) conversion of an appropriate function into a free acid;
c) conversion of an appropriate function into a pharmaceutically acceptable salt; and
d) conversion of an appropriate function into a metabolisable ester group.

The process is described in more detail in the said parent application, EP-A 58317.

The $C_1$-$C_6$ alkyl groups referred to above for $R_1$, $R_2$, $R_4$, $R_6$, $R_7$ and below for $R_{12}$, contain 1 to 6 carbon atoms and are exemplified by methyl, ethyl, propyl, butyl, pentyl, hexyl, and the corresponding branched-chain isomers thereof.

The acyl portion of the acylamino substituents referred to above for $R_1$ denotes acyl groups of the formula

$R_{12}-\overset{O}{\overset{\|}{C}}-$ wherein the group $R_{12}$ is lower alkyl, aralkyl, lower alkoxy, aryloxy, alkenyl or alkynyl of 2-6 carbon atoms, cycloalkyl of 4-6 carbon atoms, heteroaryl or heteroaralkyl, optionally substituted by hydroxy, thiol, alkylthio, lower alkyl, lower alkoxy, halogen, cyano, carboxy, nitro, amino, mono- or di-alkylamino, mono- or di-acylamino, aminoloweralkyl and/or haloloweralkyl such as trifluoromethyl. Representative of such groups are those such as benzyl, p-hydroxybenzyl, 4-amino-4-carboxybutyl, methyl, cyanomethyl, 2-pentenyl, n-amyl, n-heptyl, ethyl, 3- or 4-nitrobenzyl, phenethyl, α, β -diphenylethyl, methyldiphenylmethyl, triphenylmethyl, 2-methoxyphenyl, 2,6-dimethoxyphenyl, 2,4,6-trimethoxyphenyl, 3,5-dimethyl-4-isoxazolyl, 3-butyl-5-methyl-4-isoxazolyl, 5-methyl-3-phenyl-4-isoxazolyl, 3-(2-chlorophenyl)-5-methyl-4-isoxazolyl, 3-(2,6-dichlorophenyl), 5-methyl-4-isoxazolyl, D-4-amino-4-carboxybutyl, D-4-N-benzoylamino-4-carboxy-n-butyl, p-aminobenzyl, o-aminobenzyl, m-aminobenzyl, p-dimethylaminobenzyl, (3-pyridyl)methyl, 2-ethoxy-1-naphthyl, 3-carboxy-2-quinoxalinyl, 3-(2,6-dichlorophenyl)-5-(2-furyl)-4-isoxazolyl, 3-phenyl-4-isoxazolyl, p-carboxymethylbenzyl, m-fluorobenzyl, m-bromobenzyl, p-chlorobenzyl, p-methoxybenzyl, 1-naphthylmethyl, 3-isothiazolylmethyl, 4-isothiazolylmethyl, 5-isothiazolylmethyl, 4-pyridylmethyl, 5-isoxazolylmethyl, 4-methoxy-5-isoxazolylmethyl, 4-methyl-5-isoxazolylmethyl, 2-imidazolylmethyl, 2-benzofuranylmethyl, 2-indolylmethyl, 2-phenylvinyl, 2-phenylethynyl, 1-aminocyclohexyl, 2- and 3-thienylaminomethyl, 2-(5-nitrofuranyl) vinyl, phenyl, o-methoxyphenyl, o-chlorophenyl, o-phenylphenyl, p-aminomethylbenzyl, 1-(5-cyanotriazolyl)methyl, difluoromethyl, dichloromethyl, dibromomethyl, 1-(3-methylimidazolyl)methyl, 2- or 3-(4-carboxymethylthienyl)methyl, 2- or 3-(5-methylthienyl)methyl, 2- or 3-(methoxythienyl)methyl, 2- or 3-(4-chlorothienyl)methyl, 2- or 3-(5-carboxythienyl)methyl, 3-(1,2,5-thiadiazolyl)methyl, 3-(4-methoxy-1,2,5,-thiadiazolyl)methyl, 2-furylmethyl, 2-(5-nitrofuryl)methyl, 3-furylmethyl, 2-thienylmethyl, 3-thienylmethyl, tetrazolylmethyl, and other acyl groups found in conventional penicillin derivatives, for instance cyclohexylamidinomethyl. The term also denotes an acyl residue derived from an α-amino acid of the L or D configuration.

"Halogen" refers to a fluorine, chlorine, bromine or iodine substituent. $R_8$ as halogen is preferably fluorine.

The term "heteroaryl" as used herein for $R_4$ refers to aryl groups having a hetero atom in the ring for example, pyridyl, furanyl and thienyl. The heteroaryl group may optionally contain 1 to 3 lower alkyl substituents, e.g., 2-methylpyridyl, 3-methylthienyl. Where there is a possibility of the various position isomers, the term "heteroaryl" is intended to cover all isomers, e.g., 2-pyridyl, 3-pyridyl and 4-pyridyl.

The term "metabolisable ester" group denotes an ester group which is metabolically removed in the body. Two particularly useful metabolisable ester groups are the phthalidyl group and the pivaloyloxymethyl group.

Amine protecting groups, designated herein as N- protecting groups, and hydroxyl protecting groups, designated herein as O-protecting groups, and S-protecting groups, as well as their methods of preparation and removal are well known in the art.

Preferred N-protecting groups for use in the process of EP-A 58317 to protect amine substituents included within the definitions of $R_2$, X and Y in formula I are groups such as 2,2,2-trichloroethoxycarbonyl, benzhydryloxycarbonyl or, preferably, allyl oxycarbonyl or 2-chloroallyl.

Preferred O and S protecting groups for use in the process of EP-A 58317 are groups such as, 2,2,2-trichloroethoxycarbonyl, 1,1,1-trichloro-2-methyl-2-propoxycarbonyl, p-nitrobenzyloxycarbonyl, allyloxycarbonyl or 2-chloroallyl, with 2,2,2-trichloroethoxycarbonyl being most preferred. Suitable carboxy protecting groups are e.g. aralkyl groups conventionally used for this purpose, e.g. benzyl or p-nitrobenzyl, and allylic groups, which can be readily removed by methods known in the art, e.g. by hydrogenation in the case of aralkyl groups and e.g. by the Mc Combie method described in EP-A 13663 for the case of allylic groups.

Suitable "allylic" groups for $R_6$ (in which they perform a carboxy protective function) are any of those having the allylic structure, e.g. allyl, haloallyl, methyl allyl and crotyl. The preferred allylic group is chloroallyl, most preferably 2-chloroallyl.

The compounds of formula I possess several centres of chirality and

the process of EP-A 58317, depending on the configuration of the starting compound, will produce either chiral compounds of a specific configuration or isomeric mixtures.

The compounds of formula I may be prepared as their racemic mixtures, e.g., a 5R,6S,8R compound is produced with its enantiomer (mirror image), i.e., a 5S,6R,8S compound, in equal amounts when the starting compound of formula II is a racemic mixture. The two enantiomers may be separated by conventional means, e.g., by fractional crystallizations of optically active salt forms, e.g., the salts derived from optically active amino compounds, e.g., (-)-brucine, or (+)- and (-)-ephedrine.

Alternatively, the compounds may be produced in their pure enantiomeric forms by utilizing optically active starting materials of formula II in the synthesis procedure.

A preferred aspect of this invention is directed to compounds of formula I wherein $R_1$ is $R_4-\overset{OR_5}{\overset{|}{C}}H-$, $R_2$ is methyl or ethyl and Z and X are both sulfur, particularly the foregoing compounds wherein $R_4$ is methyl and $R_5$ is a hydroxy protecting group or preferably hydrogen. The preferred configuration of the foregoing compounds is that wherein the configuration at C-5 and C-6 is of the absolute stereochemistry R and S, respectively. The two hydrogen atoms attached to the 5 and 6 carbon atoms are thus trans to one another. The stereochemistry of the C-8 carbon atom (i.e. the carbon atom of the $R_4-\overset{OR_5}{\overset{|}{C}}H-$group) may be designated as either R or S depending on the exact nature of the $R_2$ substituent. For instance, the compounds wherein $R_4$ is methyl will have the 8R stereochemistry.

The compounds of formula I wherein $R_6$ is a metabolisable ester group can be prepared directly or by reaction of the corresponding alkali metal salt with the corresponding halide such as chlorophthalide or pivaloyloxymethyl chloride in a solvent such as dimethyl formamide. Preferably a catalytic amount of sodium iodide is added.

In the compounds of the invention, when X is $=NR_7$ it is preferred that $R_1$ is

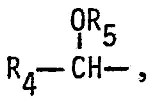 and Z is sulfur, especially when $R_2$ is lower alkyl or aminoloweralkyl.

One group of compounds within the above general formula I are compounds of the formula VII

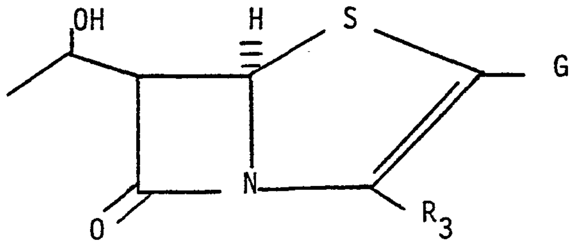

VII

in which G is loweralkoxy or the group 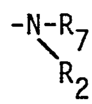 in which $R_2$, $R_3$ and $R_7$ are as defined with respect to formula I, and the pharmaceutically acceptable salts thereof.

Another group of compounds of interest is that having the general formula

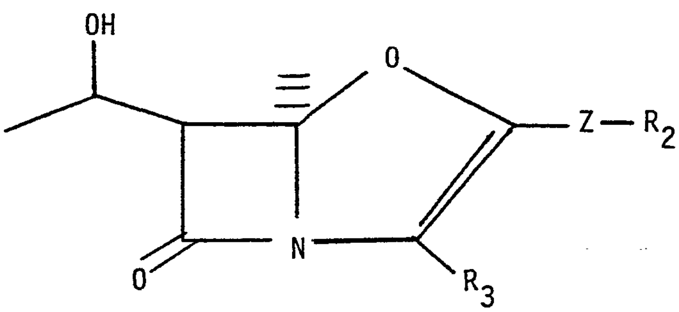

VIII

in which Z, $R_2$ and $R_3$ are as defined above with respect to formula I, $R_3$ preferably being $-COOR_6$ in which $R_6$ is as defined with respect to formula I above, and the pharmaceutically acceptable salts thereof.

Representative compounds of this invention are as follows:

(5R,6S,8R) sodium -2- (isopropylthio) -6- (1-hydroxyethyl) -2- penem -3-carboxylate,

Disodium (5R,6S,8R) -2- (3-carboxy -1- propylthio) -6- (1-hydroxyethyl) -2- penem -3- carboxylate,

Potassium (5R,6S,8R) -2- (t-butylthio) -6- (1-hydroxyethyl) -2- penem -3- carboxylate,

Sodium (5R,6S,8R) -2- ethoxy -6- (1-hydroxyethyl) -2- penem -3- carboxylate,

Sodium (5R,6S,8R) -2- butoxy -6- (1-hydroxyethyl) -2- penem -3-carboxylate.

Sodium (5R,6S,8R) -2- propoxy -6- (1-hydroxyethyl) -2- penem -3- carboxylate,

(5R,6S,8R) -2- (2 -aminoethoxy) -6- (1-hydroxyethyl) -2- penem - carboxylic acid,

Sodium (5R,6S,8R) -2- methoxy -6- (1-hydroxyethyl) -2- penem carboxylate,

(5R,6S,8R,2'S) -2- [(2'-amino-2'-carboxyethyl)thio]-6-(1-hydroxyethyl)-2-penem-3-carboxylic acid pyridinium or sodium salt,

Disodium (5R,6S,8R)-2-(2'-carboxyethylthio)-6-(1-hydroxyethyl)-2-penem-carboxylate,

Sodium (5R,6S,8R)-2-(2'-hydroxyethylthio)-6-(hydroxyethyl)-2-penem-3-carboxylate,

Sodium (5R,6S,8R)-2-(2'-ethylthio)-6-(1-fluoroethyl)-2-penem-3-carboxylate.

Certain compounds of formula I possess antibacterial activity. The antibacterially active compounds are those of the aforesaid formulae but in which, when $R_1$ contains an O, S or N protecting group and/or $R_2$ contains an O-protecting group or an N-protecting group and/or $R_7$ is an N-protecting group, then $R_3$ is chosen from nitrile, tetrazolyl, and $-COOR_6$ in

which $R_6$ is H, the radical -Alk-C(Hal)$_3$ (in which Hal is halogen and Alk represents an alkylene radical), or a metabolisable ester group; and the pharmaceutically acceptable salts thereof.

The aforementioned anti-bacterially active compounds of the invention are active against both gram-positive organisms such as Staphylococcus epidermis and Bacillus subtilis, and such gram-negative organisms as E. coli and Salmonella.

Thus the present invention includes within its scope pharmaceutical compositions comprising an antibacterially active compound or a pharmaceutically acceptable salt thereof as defined in the second preceding paragraph, together with a pharmaceutically acceptable carrier or excipient.

The dosage administered of the antibacterially active compounds of this invention is dependent upon the age and weight of the animal species being treated, the mode of administration, and the type and severity of bacterial infection being prevented or reduced. Typically, the dosage administered per day will be in the range of 5 to 200 mg/kg, with 20 to 80 mg/kg being preferred.

For oral administration, the antibacterially active compounds may be formulated in the form of tablets, capsules, elixirs or the like. Likewise, they may be admixed with animal feed. They may also be applied topically in the form of ointments, both hydrophilic and hydrophobic, in the form of lotions which may be aqueous, non-aqueous or of the emulsion type, or in the form of creams.

Also they may be utilized in liquid form such as solutions, suspensions and the like for otic and optic use and may also be administered parenterally via intramuscular injection.

The parent application, EP-A 58317, contains numerous Preparations and Examples illustrating how compounds according to the invention can be obtained, which are included herein by reference.

## CLAIMS

1. Novel penem-type compounds of the following general formula:

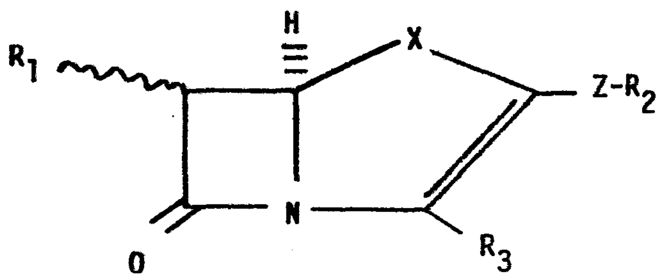

I

in which $R_1$ is hydrogen, $C_1$-$C_6$ alkyl, acylamino or $R_4\text{-}\overset{OR_5}{\overset{|}{C}}\text{H-}$, wherein $R_4$ is hydrogen, $C_1$-$C_6$ alkyl, aryl, phenyl substituted by $C_1$-$C_6$ alkyl, by $C_1$-$C_6$ alkoxy or by halogen, or heteroaryl and $R_5$ is hydrogen or an hydroxy protecting group;

$R_2$ is $C_1$-$C_6$ alkyl, aryl, phenyl substituted by $C_1$-$C_6$ alkyl, by $C_1$-$C_6$ alkoxy or by halogen, $C_1$-$C_6$ alkyl substituted by one or more aryl groups, aminoalkyl, N-protected aminoalkyl, hydroxyalkyl, O-protected hydroxyalkyl, an optionally esterfied, optionally N-protected $\alpha$-amino acid residue or an optionally esterfied carboxyalkyl group;

$R_3$ is nitrile, tetrazolyl or $-COOR_6$, wherein $R_6$ is hydrogen, $-Alk-C(Hal)_3$ (in which Hal is halogen and Alk represents a $C_1$-$C_6$ alkylene radical), $C_1$-$C_6$ alkyl, aryl, phenyl substituted by $C_1$-$C_6$ alkyl, by $C_1$-$C_6$ alkoxy or by halogen, an allylic group, an ester group which can be metabolically removed in vivo, or a carboxy protecting group;

X and Z are independently sulfur, oxygen, $-CH_2-$, $-CH_2-CH_2-$, or the radical $=NR_7$ in which $R_7$ is hydrogen, acyl, $C_1$-$C_6$ alkyl, cycloalkyl containing 3 to 6 carbon atoms, aryl, phenyl substituted by $C_1$-$C_6$ alkyl, by $C_1$-$C_6$ alkoxy or by halogen, or an N-protecting group; and wherein in the above definitions heteroaryl means an aryl group having a heteroatom in the ring and optionally having 1 to 3 $C_1$-$C_6$ alkyl substituents, and acyl refers to acyl groups derived from carboxylic acids; and the pharmaceutically acceptable salts thereof.

2. Compounds as defined in Claim 1, subject to the following provisos:

   (i) when X is S, Z is O or $=NR_7$ ($R_7$ being as defined above);

   (ii) when X is O, Z is O, S or $=NR_7$;

   (iii) when X is $-CH_2-$, Z is $=NR_7$ (except that when $R_7$ is H or $C_1$-$C_6$ alkyl and $R_2$ is $C_1$-$C_6$ alkyl, then $R_1$ cannot be acylamino);

   (iv) when X is $(CH_2)_2$, Z is $=NR_7$; and the pharmaceutically acceptable salts thereof.

3. Compounds according to Claim 2, in which X is $=NR_7$, and the pharmaceutically acceptable salts thereof.

4. Compounds according to Claim 2, having the formula:

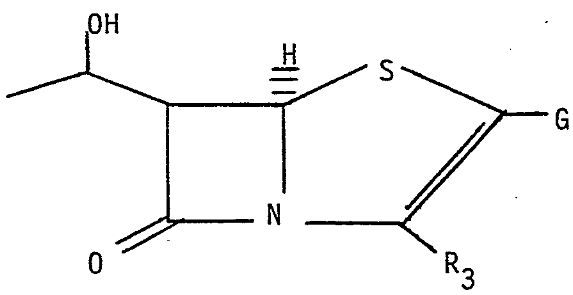

VI

in which G is $C_1$-$C_6$ alkoxy or the group $-\underset{R_2}{N}-R_7$, and wherein $R_2$, $R_3$ and $R_7$ are as defined in Claim 2; and the pharmaceutically acceptable salts thereof.

5. Compounds according to Claim 2, having the formula:

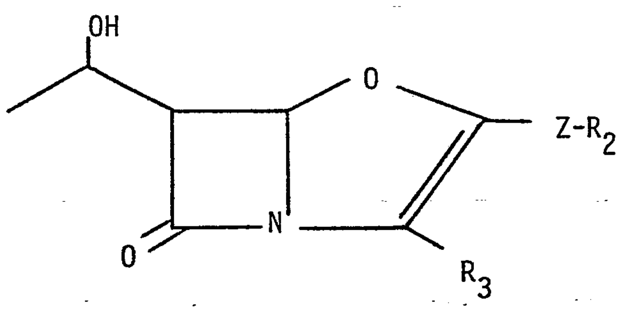

VII

in which Z, $R_2$ and $R_3$ are as defined in Claim 2, $R_3$ preferably being $-COOR_6$ in which $R_6$ is as defined in Claim 1; and the pharmaceutically acceptable salts thereof.

6. A compound as defined in any one of Claims 1 to 5, subject to the proviso that, when $R_1$ contains an hydroxyprotecting group and/or $R_2$ contains an O-protecting group or an N-protecting group, and/or $R_7$ is an N-protecting group, then $R_3$ is nitrile, tetrazolyl or $-COOR_6$ wherein $R_6$ is chosen from hydrogen, the radical $-Alk-C(Hal)_3$ (in which Hal is halogen and Alk represents an alkylene radical), or a metabolisable ester group; and the pharmaceutically acceptable salts thereof.

7. A pharmaceutical composition comprising a compound or pharmaceutically acceptable salt as defined in any of Claims 1 to 6, in admixture with a pharmaceutically acceptable carrier or excipient.

8. A compound or pharmaceutically acceptable salt as defined in any one of Claims 1 to 6, for use in the treatment of bacterial infection.

**0162193**

Application number

European Patent Office

# EUROPEAN SEARCH REPORT

EP 85 10 1420

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 172 895 (B.G. CHRISTENSEN et al.) * Claims; column 5, lines 25-28; column 5, line 51 - column 6, line 36; column 9, line 25, compound I; column 10, line 52 - column 11, line 9 * | 1,2,5, 7,8 | C 07 D 499/00<br>C 07 D 471/04<br>C 07 D 487/04<br>C 07 D 498/04<br>A 61 K 31/40<br>A 61 K 31/415<br>A 61 K 31/42<br>A 61 K 31/43<br>A 61 K 31/435//<br>(C 07 D 471/04<br>C 07 D 221:00<br>C 07 D 205:00 )<br>(C 07 D 487/04<br>C 07 D 209:00<br>C 07 D 205:00 )<br>(C 07 D 487/04<br>C 07 D 235:00<br>C 07 D 205:00 )<br>-/- |
| X | GB-A-2 042 508 (BEECHAM GROUP LTD.) * Claim 1; examples 4,5; page 2, lines 7-22; page 3, lines 46-56 * | 1,2,7, 8 | |
| D,X | EP-A-0 013 662 (SCHERING CORP.) * Claims 2-17; page 5, last line - page 6, line 16 * | 1,7,8 | |
| D,X | EP-A-0 003 960 (CIBA-GEIGY AG) * Claims 1,8-10; pages 140,141, example 88 * | 1,7,8 | **TECHNICAL FIELDS SEARCHED (Int. Cl.4)** |
| X | US-A-4 234 596 (B.G. CHRISTENSEN et al.) * Claims 2,8; abstract; column 2, line 52 - column 3, line 18, formulas; examples 12-17 * | 1,7,8 | C 07 D 471/04<br>C 07 D 487/04<br>C 07 D 498/04<br>C 07 D 499/00 |
| X | EP-A-0 004 132 (BEECHAM GROUP LTD.) * Claims 1,10,11; example 7 * --- -/- | 1,7,8 | |

The present search report has been drawn up for all claims

| Place of search | Date of completion of the search | Examiner |
|---|---|---|
| BERLIN | 19-07-1985 | HASS C V F |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

0162193

Application number

EP 85 10 1420

Page 2

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| X | EP-A-0 010 358 (GLAXO GROUP LTD.)<br>* Claims 1,2,9; page 34, formulas (V), (I) *<br>--- | 1,7,8 | (C 07 D 498/04<br>C 07 D 263:00<br>C 07 D 205:00 ) |
| P,X | EP-A-0 025 602 (KYOWA HAKKO KOGYO CO., LTD.)<br>* Claims 1,25 *<br>--- | 1,7,8 | |
| A | US-A-4 217 453 (B.G. CHRISTENSEN et al.)<br>* Examples 20,24; abstract *<br>--- | 1,2,7,<br>8 | |
| A | EP-A-0 014 476 (KYOWA HAKKO KOGYO CO., LTD.)<br>* Claims 1,23,24,27 *<br>----- | 1,7,8 | TECHNICAL FIELDS SEARCHED (Int. Cl.4) |

The present search report has been drawn up for all claims

| Place of search | Date of completion of the search | Examiner |
|---|---|---|
| BERLIN | 19-07-1985 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document